# EUROPEAN PATENT SPECIFICATION

(11) **EP 2 496 130 B1**
(45) Date of publication and mention of the grant of the patent: **30.07.2014**
(21) Application number: 10776239.5
(22) Date of filing: 02.11.2010
(51) Int. Cl.: A61B 1/04

(54) **ENDOSCOPE INCLUDING A VARIABLE STATE OPTICAL MEMBER**
ENDOSKOP MIT OPTISCHEM ELEMENT IN VARIABLEM ZUSTAND
ENDOSCOPE COMPRENANT UN ÉLÉMENT OPTIQUE À ÉTAT VARIABLE

(30) Priority: 05.11.2009 US 258422 P
(43) Date of publication of application: 12.09.2012
(73) Proprietor: Boston Scientific Scimed, Inc., Maple Grove, MN 55311-1566 (US)
(72) Inventor: DAYTON, Peter, L., Brookline, MA 02445 (US)
(74) Representative: Hauck Patent- und Rechtsanwälte
(86) International application number: PCT/US2010/055046
(87) International publication number: WO 2011/056766

(56) References cited:
- WO-A2-2009/002467
- JP-A- 11 137 512

## Description

### Field of the Invention

This invention relates to a medical device and more particularly to an endoscope having a variable state optical member that has a first configuration and a second configuration.

### Background of the Invention

Endoscopes have applications in a wide variety of medical procedures. For example, in one such procedure, an endoscope is inserted into a body lumen of a patient to obtain images of a gastrointestinal system. A variety of different types of endoscopes have various functionality. For example, some endoscopes can be used to image a body lumen with an image detector, while other endoscopes can be used to directly view a body lumen. In some instances, however, it is desirable that an endoscope can image tissue both distal and lateral to the endoscope.

A need exists for an endoscope allowing both a side view and a distal view while minimizing the number of elements within the endoscope.

Document WO2009/002467 discloses an apparatus, comprising an elongate member having a proximal end portion and a distal end portion; a variable state optical member fixed on the distal end portion of the elongate member, the variable state optical member having a first configuration and a second configuration different from the first configuration, wherein the variable state optical member is configured to pass optical energy through the variable state optical member when the variable state optical member is in the second configuration and an imager disposed within the distal end portion of the elongate member, the imager being configured to produce an imaging signal associated with a tissue distal to the elongate member when the variable state optical member is in the first configuration, and wherein the imager is further configured to produce an imaging signal associated with a tissue lateral to the elongate member when the variable State optical member is in the second configuration.

### SUMMARY OF THE INVENTION

An aspect of the present disclosure includes an apparatus which may include an elongate member having a proximal end portion and a distal end portion. The apparatus may further include a variable state optical member coupled within the distal end portion of the elongate member. The variable state optical member may have a first configuration and a second configuration different from the first configuration. The apparatus may also include an imager disposed within the distal end portion of the elongate member. The imager may be configured to produce an imaging signal associated with a tissue distal to the elongate member when the variable state optical member is in the first configuration, and the imager may be configured to produce an imaging signal associated with a tissue lateral to the elongate member when the variable state optical member is in the second configuration.

Various embodiments of the disclosure may include one or more of the following aspects: the variable state optical member may be configured to reflect optical energy when the variable state optical member is in the first configuration; the variable state optical member may be configured to pass optical energy through the variable state optical member when the variable state optical member is in the second configuration; the variable state optical member may be configured to absorb optical energy when the variable state optical member is in the first configuration; the variable state optical member may be an electrochromic mirror; the variable state optical member may have a first end portion and a second end portion opposite the first end portion, the imager and the first end portion of the variable state optical member may be separated by a first distance, and the imager and the second end portion of the variable state optical member may be separated by a second distance less than the first distance; the elongate member may define an axis, and an image plane of the imager may be substantially parallel to the axis defined by the elongate member; the distal end portion of the elongate member may define a distal aperture and a side aperture and the apparatus may further include a first lens fixedly disposed within the distal aperture of the elongate member and a second lens fixedly disposed within the side aperture of the elongate member; the variable state optical member may be configured to be moved between a first position and a second position different from the first position, and wherein the imager may be configured to produce an imaging signal associated with a tissue disposed between the tissue distal to the elongate member and the tissue lateral to the elongate member; and distal end portion may have an outer diameter less than 13 mm.

Another aspect of the present disclosure not part of the invention includes an apparatus including an elongate member which may have a proximal end portion and a distal end portion. The distal end portion of the elongate member may be configured to be inserted into a patient and may define a distal aperture and a side aperture. The apparatus may also include a first variable state optical member coupled within the distal end portion of the elongate member. The first variable state optical member may have a first configuration and a second configuration different than the first configuration. The first variable state optical member may be also configured to reflect optical energy from the distal aperture when the first variable state optical member is in its first configuration. The apparatus may also include a second variable state optical member coupled within the distal end portion of the elongate member. The second variable state optical member may have a first configuration and a second configuration different than the first configuration. The second variable state optical member may be configured to pass through optical energy from the side aperture when the second variable state optical member is in its second configuration.

Various embodiments of the present disclosure not part of the invention may include one or more of the following aspects: the first variable state optical member may be configured to pass through or absorb optical energy from the distal aperture when the first variable state optical member is in its second configuration; the second variable state optical member may be configured to absorb or reflect optical energy from the side aperture when the second variable state optical member is in its first configuration; the apparatus may further include an imager disposed within the distal end portion of the elongate member, the imager may have a first portion and a second portion different from the first portion, the first portion of the imager may be configured to receive optical energy reflected by the first variable state optical member when the first variable state optical member is in its first configuration and the second variable state optical member is in its first configuration, and the second portion of the imager may be configured to receive optical energy from the second variable state optical member when the second variable state optical member is in its second configuration and the first variable state optical member is in its second configuration; the apparatus may further include a first imager disposed within the distal end portion of the elongate member, the first imager may be configured to receive optical energy reflected by the first variable state optical member when the first variable state optical member is in its first configuration and the second variable state optical member is in its first configuration, and a second imager disposed within the distal end portion of the elongate member, the second imager may be configured to receive optical energy from the second variable state optical member when the second variable state optical member is in its second configuration and the first variable state optical member is in its second configuration; the elongate member may define an axis and the apparatus may further include an imager disposed within the distal end portion of the elongate member, wherein an image plane of the imager may be substantially parallel to the axis defined by the elongate member, the first variable state optical member may have a first end portion and a second end portion opposite the first end portion, the first end portion of the first variable state optical member and the imager may be separated by a first distance, and the second end portion of the first variable state optical member and the imager may be separated by a second distance less than the first distance; the elongate member may define an axis and the second variable state optical member may be parallel to the axis defined by the elongate member; the side aperture may be a first side aperture and the distal end portion of the elongate member may define a second side aperture different from the first side aperture, wherein the apparatus may further include a third variable state optical member coupled within the distal end portion of the elongate member, the third variable state optical member may have a first configuration and a second configuration different than the first configuration, the third variable state optical member may be configured to reflect optical energy from the second side aperture when the third variable state optical member in its first configuration, and the third variable state optical member may be configured to pass through optical energy from the second side aperture when the third variable state optical member is in its second configuration; the first variable state optical member may be an electrochromic mirror; the distal end portion of the elongate member may define a third aperture between the distal aperture and the side aperture, the first variable state optical member includes a first position and a second position different from the first position, and the first variable state optical member may be configured to receive optical energy from the third aperture when in the second position; and the distal end portion may have a width less than 13 mm.

Another aspect of the present disclosure not part of the invention may include a method of sending an imaging signal associated with a tissue distal to an elongate medical device while a variable state optical member of the elongate medical device is in a first configuration. The method may further include changing the variable state optical member from the first configuration to a second configuration after sending the imaging signal while the variable state optical member is in the first configuration. Additionally, the method may include sending an imaging signal associated with a tissue lateral to the elongate medical device while the variable state optical member is in the second configuration.

Various embodiments of the present disclosure not part of the invention may include one or more of the following aspects: the variable state optical member may be a first variable state optical member and the method may further include changing a second variable state optical member of the elongate medical device from a first configuration to a second configuration, wherein the imaging signal associated with the tissue distal to the elongate medical device may be sent while the first variable state optical member is in the first configuration and the second variable state optical member is in the second configuration, and the imaging signal associated with the tissue lateral to the elongate medical device may be sent while the first variable state optical member is in the second configuration and the second variable state optical member is in the first configuration; the method may further include moving the variable state optical member from a first position to a second position and sending an imaging signal associated with a tissue distal and lateral to the elongate medical device; and the variable state optical member may be a first variable state optical member and the method may further include moving a second variable state optical member of the elongate medical device from a first position to a second position.

A further aspect of the present disclosure not part of the invention includes an apparatus including an elongate member having a proximal end portion and a distal end portion. The apparatus may also include a first variable state optical member coupled within the distal end portion of the elongate member. The first variable state optical member may have a transmissive configuration and a non-transmissive configuration. The apparatus may also include a second variable state optical member coupled within the distal end portion of the elongate member. The second variable state optical member may have a transmissive configuration and a non-transmissive configuration. Additionally, the apparatus may include an imager disposed within the distal end portion of the elongate member. The imager may be configured to produce an imaging signal associated with a tissue distal to the elongate member and an imaging signal associated with a tissue lateral to the elongate member, wherein the imager may define an image plane diagonal to a centerline defined by the distal end portion of the elongate member.

Various embodiments of the present disclosure not part of the invention may include one or more of the following aspects: the imager may be configured to produce the imaging signal associated with the tissue distal to the elongate member when the first variable state optical member is in its transmissive configuration and the second variable state optical member is in its non-transmissive configuration, and the imager may be configured to produce the imaging signal associated with the tissue lateral to the elongate member when the first variable state optical member is in its non-transmissive configuration and the second variable state optical member is in its transmissive configuration; the imager may include a first portion and a second portion mutually exclusive from the first portion, the first portion of the imager may be configured to produce the imaging signal associated with the tissue distal to the elongate member when the first variable state optical member and the second variable state optical member are each in their respective transmissive configurations, and the second portion of the imager may be configured to produce the imaging signal associated with the tissue lateral to the elongate member when the first variable state optical member and the second variable state optical member are each in their respective transmissive configurations; and the first variable state optical member is substantially perpendicular to the centerline and the second variable state optical member may be substantially parallel to the centerline.

Additional objects and advantages of the invention will be set forth in part in the description which follows, and in part will be obvious from the description, or may be learned by practice of the invention. The objects and advantages of the invention will be realized and attained by means of the elements and combinations particularly pointed out in the appended claims.

It is to be understood that both the foregoing general description and the following detailed description are exemplary and explanatory only and are not restrictive of the invention, as claimed.

The accompanying drawings, which are incorporated in and constitute a part of this specification, illustrate several embodiments of the invention and together with the description, serve to explain the principles of the invention.

### BRIEF DESCRIPTION OF THE DRAWINGS

FIG. 1 is a schematic illustration of a medical device according to an embodiment of the invention.
FIG. 2 is a distal end view of a medical device according to an embodiment of the invention.
FIG. 3 is a side view of a portion of the medical device of FIG. 2.
FIGS. 4 and 5 are cross-sectional views of a portion of the medical device of FIG. 2 taken along line 4-4 in FIG. 2.
FIGS. 6 and 7 are cross-sectional views of a portion of a medical device according to an embodiment not part of the invention.
FIG. 8 is a cross-sectional view of a portion of a medical device according to an embodiment not part of the invention.
FIG. 9 is a flowchart illustrating a process of a medical device according to an embodiment not part of the invention.
FIG. 10 is a side perspective view of a medical device shown operatively coupled to a visual display.

### DESCRIPTION OF THE EMBODIMENTS

Reference will now be made in detail to the present embodiments (exemplary embodiments) of the invention, examples of which are illustrated in the accompanying drawings. Wherever possible, the same reference numbers will be used throughout the drawings to refer to the same or like parts.

The medical devices described herein can be used in a body of a patient including, for example, a variety of different body cavities or lumens such as, for example, a gastrointestinal lumen, a vascular lumen, a ureteral lumen, a duodenum, etc. The medical device can also be inserted into a portion of a patient via an incision (e.g., an aperture) within the skin or within a body lumen. As described herein, a medical device can include a detector disposed at a distal end portion of the medical device. The detector can comprise, for example, an imaging device such as a charge coupled device (CCD) or a complementary metal-oxide-semiconductor (CMOS) sensor. The detector can be configured to receive optical energy, (sometimes referred to herein as electromagnetic energy), such as, for example, optical energy reflected from a tissue located at or near a distal end portion of the medical device and can generate an electrical signal (e.g., an imaging signal). The electrical signal can then be transmitted to a proximal end portion of the medical device to, or remotely to, a processor or display monitor.

Using a variable state optical member having a first configuration and a second configuration can reduce the number of elements of the medical device, and thus can also allow for a reduction in the size of the medical device. For example, in some embodiments, a medical device (also referred to herein as an endoscope) can direct optical energy towards a detector via a variable state optical member. The variable state optical member can have an optically-reflective configuration (e.g., a mirror configuration or an absorptive configuration) and an optically-transmissive configuration (e.g., a transparent configuration). A first set of optical energy received by the endoscope can be directed towards the detector via the variable state optical member of the medical device when the variable state optical member is in an optically-reflective configuration. The variable state optical member can also inhibit a second set of optical energy from being received by the detector when the variable state optical member is in the optically-reflective configuration. The second set of optical energy and not the first set of optical energy can be received by the detector when the variable state optical member is in the optically passive-configuration. Thus, the detector can receive two independent sets of optical energy based on the configuration of the variable state optical member of the endoscope.

The variable state optical member can be, for example, a variable transmittance/reflectance device. The variable state optical member can reversibly switch among an optically-reflective or mirror-like configuration, an optically-transmissive or transparent configuration, and/or an optically-absorptive configuration. For example, the variable state optical member can change between the configurations based on electrochromic or gasochromic means. Specifically, the variable state optical member can exhibit a mirror-like configuration when no electric field or hydrogen is present. The variable state optical member can change to a transparent configuration when an electric field or hydrogen is applied to the variable state optical member or vice versa.

In one embodiment, an endoscope includes an elongate member having a proximal end portion and a distal end portion. A variable state optical member can be coupled within the distal end portion of the elongate member. The variable state optical member has a first configuration and a second configuration different from the first configuration. A detector can be disposed within the distal end portion of the elongate member. The detector is configured to produce an imaging signal associated with a tissue distal to the elongate member when the variable state optical member is in a first configuration. The detector is configured to produce an imaging signal associated with a tissue lateral to the elongate member when the variable state optical member is in a second configuration.

The terms "proximal" and "distal" refer to direction closer to and away from, respectively, an operator (e.g., surgeon, physician, nurse, technician, etc.) who would insert the medical device into the patient, with the tip-end (i.e., distal end) of the device inserted inside a patient's body. Thus, for example, the endoscope end inserted inside a patient's body would be the distal end of the endoscope, while the endoscope end outside a patient's body would be the proximal end of the endoscope.

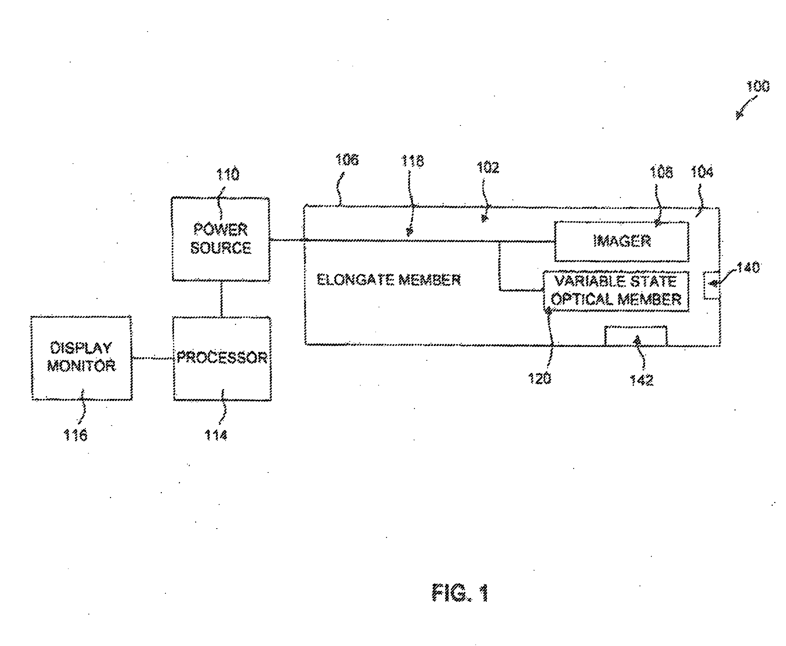

Figure 1 is a schematic illustration of a medical device according to an embodiment of the invention. A medical device 100 (also referred herein as an "endoscope") includes an elongate member 102 having a proximal end portion 106 and a distal end portion 104. The distal end portion 104 can be inserted at least partially into a patient. The distal end portion 104 of the elongate member 102 can define a distal aperture 140 and a side aperture 142. An imager 108 can be coupled within the distal end portion 104 of the elongate member 102. For example, the imager 108 can be disposed within a lumen (not shown) of the distal end portion 104 of the elongate member 102. The imager 108 can be configured to receive optical energy and can produce an electrical signal in response. The imager 108 can be operatively coupled to a display monitor 116 that can display an image based on the imaging signals. The image is associated with at least a portion of a tissue of the patient. The imager 108 can also be coupled to a processor 114. An elongate conductive component 118 (e.g., a conductive wire) can be electrically coupled to the imager 108 and a power source 110. The elongate conductive component 118 can transmit electrical power from the power source 110 to the detector 108. In some embodiments, a second elongate conductive component (not shown) can be coupled to the detector 108 and the display monitor 116. The second elongate conductive component can transmit a signal (e.g., the imaging signal) produced by the detector 108 to the display monitor 116. In some embodiments, information may be relayed remotely, through the body, using, for example, radio communication between the detector 108 and the processor 114 (e.g., a controller external to the body).

A variable state optical member 120 can be disposed within the distal end portion 104 of the elongate member 102. The variable state optical member 120 can have a first configuration (e.g., an optically-non-transmissive configuration, an optically- reflective configuration) and a second configuration (e.g., an optically-transmissive configuration) different from the first configuration. The imager 108 can produce an imaging signal associated with a tissue distal to the elongate member 102 when the variable state optical member 120 is in a first configuration and the distal end portion 104 is disposed within a patient. In other words, the imager 108 can receive optical energy from the distal aperture 140 when the variable state optical member 120 is in a first configuration. The imager 108 can produce an imaging signal associated with a tissue lateral to the elongate member 102 when the variable state optical member 120 is in a second configuration and the distal end portion 104 is disposed within the patient. Said differently, the imager 108 can receive optical energy from the side aperture 142 when the variable state optical member 120 is in a second configuration. In some embodiments, the variable state optical member 120 can optionally have more or less than the configurations described above.

In some embodiments, the variable state optical member can move between a first position and a second position different from the first position. The first position of the variable state optical member can be a diagonal position with respect to the imager such that the variable state optical member manipulates optical energy as described above. The variable state optical member can be positioned in the second position such that the imager can produce an imaging signal associated with a tissue between the tissue distal to the elongate member and the tissue lateral to the elongate member. In some embodiments, the variable state optical member can have more or less than the two positions described above. In some embodiments, the variable state optical member can be positioned to form one or more angles with the imager.

Although FIG. 1 shows one variable state optical member, in some alternative embodiments not part of the invention, the medical device includes a second variable state optical member (or more than two variable state optical members). Similarly, the medical device in some embodiments includes a second detector (or more than two detectors). In some embodiments, one reflector may reflect two separate images to two separate detectors.

In some embodiments, the distal end portion has an outer diameter less than 13 mm. In other embodiments, a width of the detector is less than 13 mm.

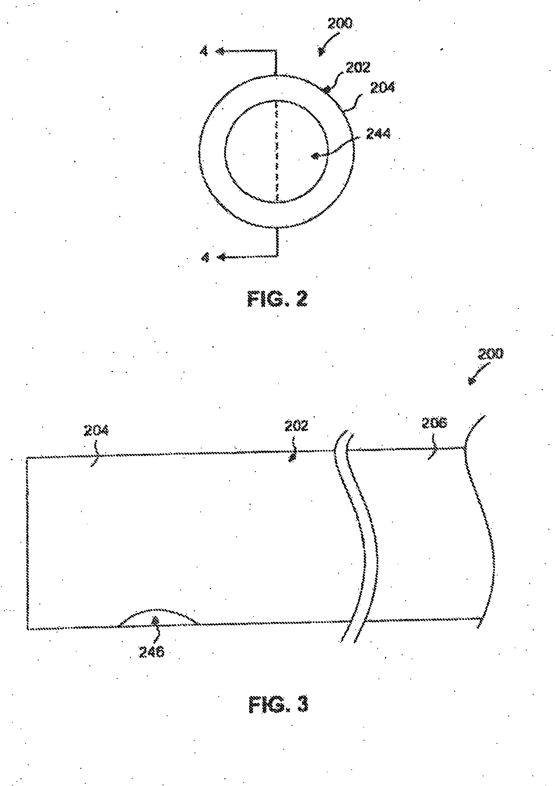

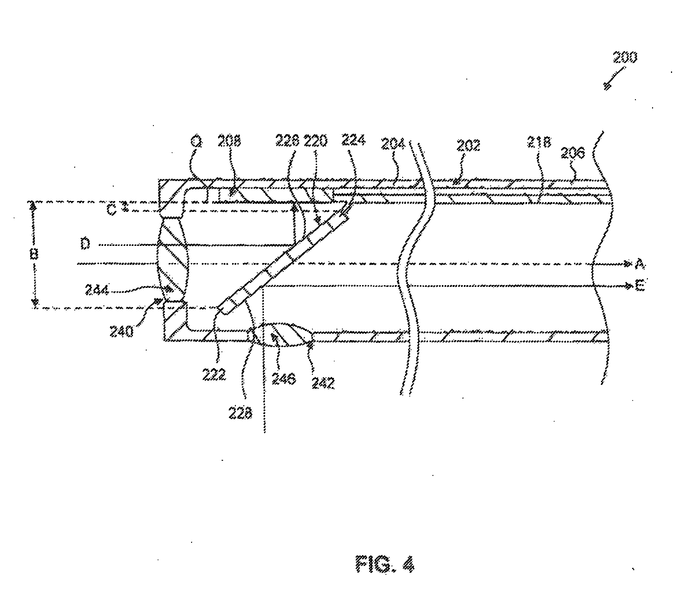

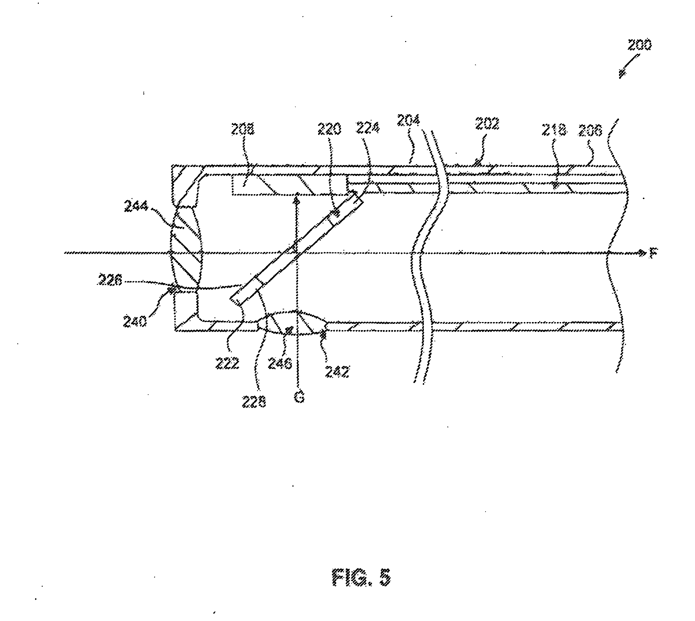

FIGS. 2 - 5 illustrate a medical device according to an embodiment of the invention. As illustrated in FIGS. 4 and 5, an endoscope 200 may include an elongate member 202 having a distal end portion 204 and a proximal end portion 206. The elongate member 202 defines an axis A (sometimes referred to herein as a centerline). An imager 208 can be disposed within the distal end portion 204 of the elongate member 202. The imager 208 has an imaging plane Q that is substantially parallel to the axis A. The imager 208 can receive optical energy and produce an imaging signal based on the optical energy. The imaging signal may include information associated with an image. In use, the image is based on optical energy reflected from at least a portion of a tissue of a patient. The imager 208 can be any of a variety of different imaging devices, including for example, a charge coupled device (CCD), a complementary metal-oxide-semiconductor (CMOS) sensor, an active pixel sensor, a thermal imaging sensor, a video camera tube, a gamma camera sensor, a x-ray sensor, or the like. The proximal end portion 206 of the elongate member 202 can be operatively coupled to a visual display (not shown). In this embodiment, the imager 208 is substantially planar (e.g., the imager 208 has a substantially rectangular shape). In an alternative embodiment, the imager can have a non-planar and/or non-rectangular shape.

In some embodiments, optical energy can be transmitted from an optical source (not shown), via an elongate optical component (e.g., an optical fiber not shown), to illuminate tissue located at or near the distal end portion 204 of the elongate member 202. The imager 208 is configured to receive optical energy reflected from the tissue and to produce an imaging signal based on the optical energy reflected from the tissue located at or near the distal end portion 204 of the elongate member 202. In some embodiments, the optical source is integrated with the endoscope. In other embodiments, the optical source is separate from the endoscope.

An elongate conductive component 218 can be electrically coupled to the imager 208 and a power source (not shown). The elongate conductive component 218 can transmit electrical power from the power source to the imager 208. The endoscope 200 can include a second elongate conductive component (not shown) coupled to the imager 208 and a visual display (not shown). The second elongate conductive component can transmit an imaging signal produced by the imager 208 to the visual display such that an image associated with the imaging signal is displayed by the visual display. In some embodiments, the second elongate conductive component can be coupled to a processor (not shown) disposed at the proximal end portion 206 of the elongate member 202. The second elongate conductive component can transmit an imaging signal from the imager 208 to the processor. In some embodiments, information is transferred between the imager and processor remotely, without the use of wires.

The distal end portion 204 of the elongate member 202 may define a distal aperture 240 and a side aperture 242. A first lens 244 can be fixedly disposed within the distal aperture 240 of the distal end portion 204 of the elongate member 202. A second lens 246 can be fixedly disposed within the side aperture 242 of the distal end portion 204 of the elongate member 202. The first lens 244 and the second lens 246 can each be hermetically sealed to their respective apertures 240 and 242 to inhibit fluids or tissue from entering the elongate member 202. The first lens 244 and the second lens 246 can each have one of a variety of different lens configurations, including for example, biconvex, plano-convex, convex-concave, meniscus, plano-concave, biconcave, flat, etc. The imaging device 208 can produce an image focused by the first lens 244 or the second lens 246 so that the image can be output to a processor or visual display as described previously. A first focal plane and a second focal plane are defined by the first lens 244 and the second lens 246, respectively. Each lens configuration defines, at least in part, the position of the focal plane. The lens configuration of the first lens 244 and the second lens 246 can each define, at least in part, the magnification of the image produced by the imager 208. In some embodiments, the distal end portion 204 of the elongate member 202 defines more or less than two apertures. In some embodiments, more or less than two lenses can optionally be used. Specifically, more lenses can be used within the distal aperture 240 and within the side aperture 242.

The endoscope 200 can include a variable state optical member 220 fixedly coupled within the distal end portion 204 of the elongate member 202. The variable state optical member 220 can be diagonally positioned within the distal end portion 204 of the elongate member 202 such that optical energy can be reflected towards the imager 208. Specifically, the variable state optical member 220 has a distal end portion 222 and a proximal end portion 224. The distal end portion 222 of the variable state optical member 220 and the imager 208 is separated by a distance B. The proximal end portion 224 of the variable state optical member 220 and the imager 208 is separated by a distance C. The distance B is greater than the distance C. The variable state optical member 220 can have one of a variety of different configurations, including for example, biconvex, plano-convex, convex-concave, meniscus, plano-concave, biconcave, flat, etc.

The variable state optical member 220 can have a first configuration and a second configuration different from the first configuration. The variable state optical member 220 reflects optical energy and inhibits the passage of optical energy through the variable state optical member 220 when the variable state optical member 220 is in the first configuration. The variable state optical member 220 permits optical energy to pass through the variable state optical member 220 when the variable state optical member 220 is in the second configuration. Specifically, the variable state optical member 220 has a first side portion 226 and a second side portion 228 opposite the first side 226 portion. The first side portion 226 and the second side portion 228 of the variable state optical member 220 can each reflect optical energy when the variable state optical member 220 is in the first configuration. The variable state optical member 220 passes optical energy between the first side portion 226 and the second side portion 228 when the variable state optical member 220 is in the second configuration.

The variable state optical member 220 can change between the first configuration and the second configuration. Specifically, the variable state optical member 220 can change from the first configuration to the second configuration when, for example, electrical power is applied, via the elongate conductive component 218, to the variable state optical member 220. In some embodiments, remote control is allowed where there is no need for the elongate conductive component 218. The variable state optical member 220 can change from the second configuration to the first configuration when, for example, electrical power is removed from (e.g., not applied to) the variable state optical member 220. In this embodiment, although electrical power applied to the variable state optical member 220 changes the configuration of the variable state optical member 220, it should be understood that the configuration of the variable state optical member 220 can be changed by any of a variety of different mechanisms including for example, a gasochromic mechanism, or a mechanical device, such as a motor, a shape changing or electroactive material including metal, polymer, or gel.

The imager 208 can produce an imaging signal associated with a tissue (not shown) distal to the elongate member 202 when the variable state optical member 220 is in the first configuration. Optical energy received from the first lens 244 and the second lens 246 travels along optical paths D and E, respectively, when the variable state optical member 220 is in the first configuration (e.g., a reflective configuration) as shown in FIG. 4. In other words, optical energy received from the first lens 244 (e.g., a distal lens) is reflected at the variable state optical member 220 towards the imager 208. Accordingly, the imager 208 can produce an imaging signal based on optical energy reflected from at least a portion of the tissue distal to the distal end portion 204 of the elongate member 202. Optical energy received from the second lens 246 (e.g., a side lens) is reflected at the variable state optical member 220 away from the imager 208 such that the optical energy is not received by the imager 208. Accordingly, an imaging signal produced by the imager 208 is not based on optical energy reflected from the tissue lateral to the distal end portion 204 of the elongate member 202.

The imager 208 can produce an imaging signal associated with a tissue (not shown) lateral to the elongate member 202 when the variable state optical member 220 is in the second configuration. Optical energy received from the first lens 244 and the second lens 246 travels along optical paths F and G, respectively, when the variable state optical member 220 is in the second configuration (e.g., a pass-through configuration) as shown in FIG. 5. In other words, optical energy received from the first lens 244 (e.g., the distal lens) can pass through the variable state optical member 220 and does not contact the imager 208. Accordingly, an imaging signal produced by the imager 208 is not based on optical energy reflected from the tissue distal to the distal end portion 204 of the elongate member 202. Optical energy received from the second lens 246 (e.g., the side lens) can pass through the variable state optical member 220 and is received by the imager 208. Accordingly, the imager 208 can produce an imaging signal based on optical energy reflected from at least a portion of a tissue lateral to the distal end portion 204 of the elongate member 202.

In an alternative embodiment, the image plane of the imager can be perpendicular to the longitudinal axis or centerline defined by the elongate member. In such an embodiment, the imager can produce an imaging signal associated with a tissue lateral to the elongate member when the variable state optical member is in the first configuration (e.g., an optically reflective configuration). The imager can produce an imaging signal associated with a tissue distal to the elongate member when the variable state optical member is in the second configuration (e.g., an optically-passive configuration). In this embodiment, the variable state optical member is fixedly coupled within the distal end portion of the elongate member.

In yet another embodiment, the variable state optical member can be pivotally, slidably, or movably coupled within the distal end portion of the elongate member. Said differently, the variable state optical member can move between a first position, similar to that shown in FIG. 4, and a second position (not shown) different from the first position. The imager can produce an imaging signal based on optical energy reflected by at least a portion of a tissue disposed between the tissue distal to the elongate member and the tissue lateral to the elongate member when the variable state optical member is in the second position. Alternatively, the variable state optical member can have more than two positions.

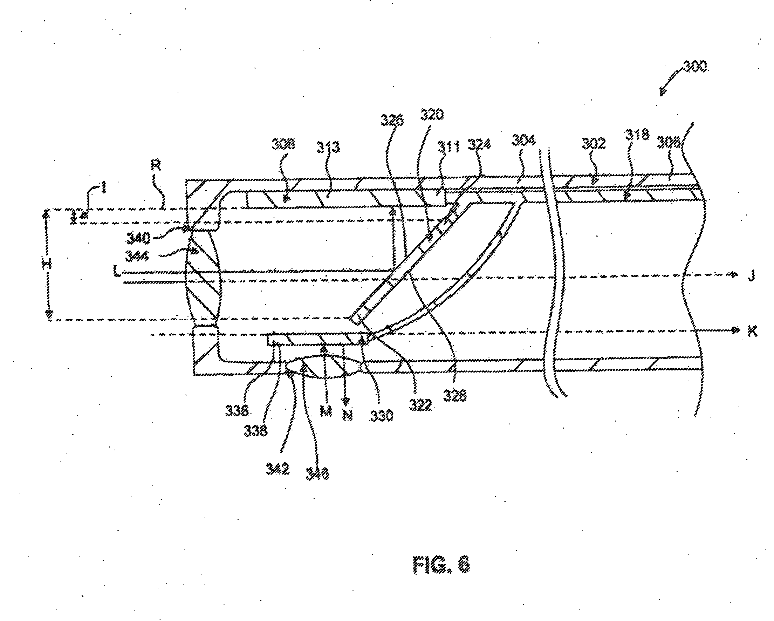

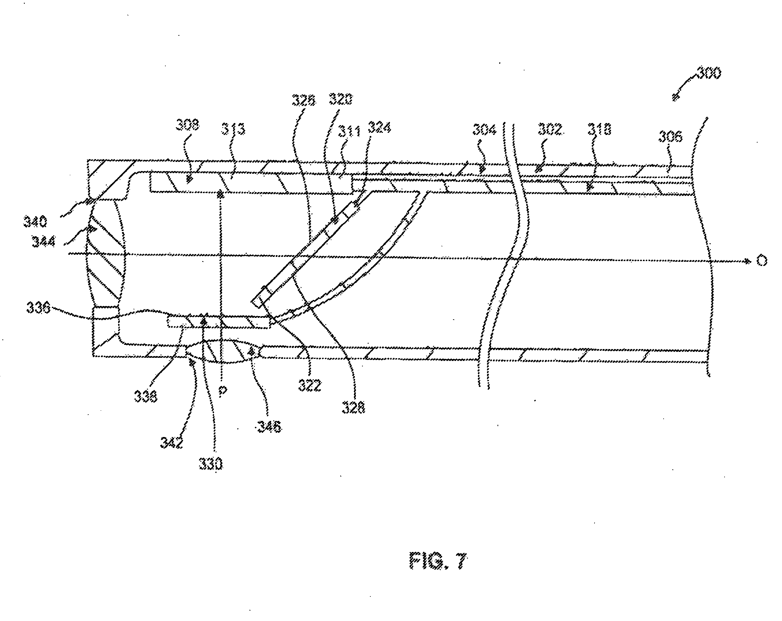

FIGS. 6 and 7 illustrate a medical device according to another embodiment not part of the invention. As shown in FIGS. 6 and 7, an endoscope 300 may include an elongate member 302 having a distal end portion 304 and a proximal end portion 306. The distal end portion 304 of the elongate member 302 can be inserted into a patient. The distal end portion 304 of the elongate member 302 defines a distal aperture 340 and a side aperture 342. The distal end portion 304 of the elongate member 302 includes a first lens 344 and a second lens 346 fixedly disposed within the distal aperture 340 and the side aperture 342, respectively. An imager 308 is disposed within the distal end portion 304 of the elongate member 302. The elongate member 302 defines an axis or centerline J. An image plane R of the imager 308 is substantially parallel to the axis J defined by the elongate member 302.

As with the previous embodiment not part of the invention, an elongate conductive component 318 is electrically coupled to the imager 308 and can transmit electrical power from a power source (not shown) to the imager 308. Alternatively, electrical power can be transmitted from the power source to the imager 308 remotely. A second elongate conductive component (not shown) can be coupled to the imager 308. The second elongate conductive component can transmit a signal produced by the imager 308 to a processor (not shown) and/or a display monitor (not shown) disposed at the proximal end portion 306 of the elongate member 302. Alternatively, the signal can be transmitted to the processor remotely.

In this embodiment not part of the invention, a first variable state optical member 320 and a second variable state optical member 330 are each fixedly disposed within the distal end portion 304 of the elongate member 302. The first variable state optical member 320 is diagonally positioned within the distal end portion 304 of the elongate member 302. Specifically, the first variable state optical member 320 has a distal end portion 322 and a proximal end portion 324. The distal end portion 322 of the first variable state optical member 320 and the imager 308 is separated by a distance H. The proximal end portion 324 of the first variable state optical member 320 and the imager 308 is separated by a distance I. The distance H is greater than the distance I. The second variable state optical member 330 is positioned in a parallel arrangement with respect to the elongate member 302. Specifically, the second variable state optical member 330 defines an axis K substantially parallel to axis J. More specifically, the first variable state optical member 320 is positioned to receive optical energy received from the first lens 344. The second variable state optical member 330 is positioned to receive optical energy from the second lens 346. The first variable state optical member 320 and the second variable state optical member 330 can each have one of a variety of different configurations, including for example, biconvex, plano-convex, convex-concave, meniscus, planoconcave, biconcave, flat, etc.

The first variable state optical member 320 has a first configuration and a second configuration different from the first configuration. The first variable state optical member 320 can reflect optical energy towards the imager 308 when the first variable state optical member 320 is in the first configuration. The first variable state optical member 320 can permit optical energy to pass through the first variable state optical member 320 and away from the imager 308 when the first variable state optical member 320 is in the second configuration. Specifically, the first variable state optical member 320 has a first side portion 326 and a second side portion 328 opposite the first side 326 portion. The first side portion 326 of the first variable state optical member 320 reflects optical energy when the first variable state optical member 320 is in the first configuration. The first variable state optical member 320 passes optical energy between the first side portion 326 of the first variable state optical member 320 and the second side portion 328 of the first variable state optical member 320 when the first variable state optical member 320 is in the second configuration. In an alternative embodiment, the first variable state optical member absorbs optical energy when the first variable state optical member 320 is in the second configuration.

Similarly, the second variable state optical member 330 can have a first configuration (e.g., an optically-transmissive configuration, an optically-reflective configuration or an optically-absorptive configuration) and a second configuration (e.g., an optically-transmissive configuration) different from the first configuration. The second variable state optical member 330 can reflect optical energy to inhibit the passage of optical energy when the second variable state optical member 330 is in the first configuration. Said differently, the second variable state optical member 330 inhibits optical energy reflected from tissue lateral to the elongate member 302 from being received by the imager 308 when the second variable state optical member 330 is in the first configuration. The second variable state optical member 330 permits optical energy to pass through the second variable state optical member 330 when the second variable state optical member 330 is in the second configuration. Said differently, the second variable state optical member 330 has a first side portion 336 and a second side portion 338 opposite the first side portion 336. The second side portion 338 of the second variable state optical member 330 reflects optical energy when the second variable state optical member 330 is in the second configuration. The second variable state optical member 330 passes optical energy between the first side portion 336 of the second variable state optical member 330 and the second side portion 338 of the second variable state optical member 330 when the second variable state optical member 330 is in the second configuration. In an alternative embodiment, the second variable state optical member 330 can absorb optical energy to inhibit the passage of optical energy when the second variable state optical member 330 is in the first configuration.

The elongate conductive component 318 is electrically coupled to the first variable state optical member 320 and the second variable state optical member 330. The elongate conductive component 318 can transmit electrical power from the power source to the first variable state optical member 320 and the second state optical member 330. In some embodiments, the first variable state optical member 320 and the second variable state optical member 330 receive electrical power from the power source via separate electrical conductive components.

The first variable state optical member 320 can change between the first configuration and the second configuration. Specifically, the first variable state optical member 320 can change from the first configuration to the second configuration when electrical power, via the elongate conductive component 318, is applied to the first variable state optical member 320. The first variable state optical member 320 can change from the second configuration to the first configuration when electrical power is removed from (e.g., not applied to) the first variable state optical member 320. Although in this embodiment, the electrical power applied to the first variable state optical member 320 changes the configuration of the first variable state optical member 320, it should be understood that the configuration of the first variable state optical member 320 can be changed by any of a variety of different mechanisms, including for example, a gasochromic mechanism, a mechanical device, a thermal device, etc. In some embodiments, the first variable state optical member 320 and/or the second variable state optical member 330 can optionally have more than two configurations. The first variable state optical member 320 and/or the second variable state optical member 330 can be, for example, a thermochromic mirror, a photochromic mirror, an electrochromic mirror, a solvatochromic mirror, a gasochromic mirror or the like.

Similarly, the second variable state optical member 330 can change between the first configuration and the second configuration. Specifically, the second variable state optical member 330 can change from the first configuration to the second configuration when electrical power is applied, via the elongate conductive component 318, to the second variable state optical member 330. The second variable state optical member 330 can change from the second configuration to the first configuration when electrical power is removed from (e.g., not applied to) the second variable state optical member 330. Although in this embodiment, the electrical power applied to the second variable state optical member 330 changes the configuration of the second variable state optical member 330, it should be understood that the configuration of the second variable state optical member 330 can be changed by any of a variety of different means, including for example, gasochromic means, a mechanical device, see examples above.

In this embodiment not part of the invention, the imager 308 includes a first portion 311 and a second portion 313. The first portion 311 of the imager 308 can receive optical energy reflected by the first variable state optical member 320 when the first variable state optical member 320 is in its first configuration and the second variable state optical member 330 is in its first configuration. The second portion 313 of the imager 308 can receive optical energy from the second variable state optical member 330 when the second variable state optical member 330 is in its second configuration and the first variable state optical member 320 is in its second configuration. In other words, the first portion 311 of the imager 308 can produce an imaging signal independent of the second portion 313 of the imager 308. Similarly, the second portion 313 of the imager 308 can produce an imaging signal independent of the first portion 311 of the imager 308. Note that the distal portion of the imager 308 captures the image for the tissue lateral to the elongate member 302; the proximal end portion of the imager 308 captures the image for the tissue distal to the elongate member 302.

The imager 308 can produce an imaging signal associated with a tissue (not shown) distal to the elongate member 302 when the first variable state optical member 320 is in its first configuration and the second variable state optical member 330 is in its first configuration. Optical energy received from the first lens 344 travels along optical paths L when the first variable state optical member 320 is in the first configuration (e.g., a reflective configuration) as shown in FIG. 6. In other words, optical energy received from the first lens 344 (e.g., a distal lens) is reflected at the first variable state optical member 320 towards the first portion 311 of the imager 308. Accordingly, the first portion 311 imager 308 can produce an imaging signal associated with an image of at least a portion of the tissue distal to the distal end portion 304 of the elongate member 302. Optical energy received at the second lens 346 (e.g., a side lens), as shown by optical path M, is absorbed or reflected at the second variable state optical member 330 away from the imager 308, shown by optical path N, such that the optical energy is not received by the imager 308 when the second variable state optical member 330 is in the first configuration. Accordingly, the imaging signal produced by the imager 308 is not associated with an image of the tissue lateral to the distal end portion 304 of the elongate member 302.

The imager 308 can produce an imaging signal associated with a tissue (not shown) lateral to the elongate member 302 when the first variable state optical member 320 is in its second configuration and the second variable state optical member 330 is in its second configuration. Optical energy received from the first lens 344 and the second lens 346 travels along optical paths O and P, respectively, when the first variable state optical member 320 is in its second configuration (e.g., a pass-through configuration) and the second variable state optical member 330 is in its second configuration (e.g., a pass-through configuration) as shown in FIG. 7. In other words, optical energy received from the first lens 344 can pass through the first variable state optical member 320 and is not received at the imager 308. Accordingly, the imaging signal produced by the imager 308 does not include optical energy reflected from the tissue distal to the elongate member 302. Optical energy received from the second lens 346 can pass through the second variable state optical member 330 and is received by the second portion 313 of the imager 308. Accordingly, the second portion 313 of the imager 308 can produce an imaging signal associated with an image of at least a portion of a tissue lateral to the elongate member 302. In this embodiment, the first variable state optical member 320 and the second variable state optical member 330 are both fixedly coupled within the distal end portion 304 of the elongate member 302. In some embodiments, the imager can concurrently produce both an imaging signal associated with tissue distal to the elongate member and an imaging signal associated with tissue lateral to the elongate member. In such embodiments, the first portion of the imager can produce the imaging signal associated with tissue distal to the elongate member when the first variable state optical member is in its first configuration (e.g., a reflective configuration). The second portion of the imager can concurrently produce an imaging signal associated with tissue lateral to the elongate member when the second variable state optical member is in its second configuration (e.g., a pass-through configuration).

In an alternative embodiment not part of the invention, the imager is a first imager. A second imager (not shown) is fixedly coupled within the distal end portion of the elongate member. The second imager defining an imaging plane substantially parallel to the axis defined by the elongate member. In such an embodiment, the first imager can receive optical energy reflected by the first variable state optical member when the first variable state optical member is in its first configuration and the second variable state optical member is in its first configuration. The second imager can receive optical energy from the second variable state optical member when the second variable state optical member is in its second configuration and the first variable state optical member is in its second configuration. In other words, optical energy is reflected away from the second imager when the second variable state optical member is in the first configuration. Thus, the image produced by the second imager is not based on optical energy received from the second variable state optical member when the second variable state optical member is in its first configuration. Optical energy can be received by the second imager when the second variable state optical member is in the second configuration. Accordingly, the second imager can produce an image signal based on the optical energy when the second variable state optical member is in the second configuration.

In an alternative embodiment not part of the invention, the first variable state optical member and/or the second variable state optical member can be pivotally, slidably, or movably coupled within the distal end portion of the elongate member. Said differently, the first variable state optical member and/or second variable state optical member can move between a first position, similar to that shown in FIG. 6, and a second position (not shown) different from the first position. The distal end portion of the elongate member can define a third aperture between the distal aperture and the side aperture. For example, the third aperture can have an elongate, slot shape between the distal aperture and the side aperture. The imager can produce an imaging signal associated with an image of at least a portion of a tissue disposed between the tissue distal to the elongate member and the tissue lateral to the elongate member when at least one of the first variable state optical member or the second variable state optical member is in the second configuration.

In some embodiments not part of the invention, the distal end portion of the elongate member can define a second side aperture (not shown). A third variable state optical member (not shown) can be fixedly coupled within the distal end portion of the elongate member. The third variable state optical member has a first configuration and a second configuration different than the first configuration. The third variable state optical member can reflect optical energy from the second side aperture when the third variable state optical member is in its first configuration. The third variable state optical member can pass through optical energy from the second side aperture when the third variable state optical member is in its second configuration.

In an alternative embodiment not part of the invention, the imager is a first imager. The first variable state optical member is substantially perpendicular to the longitudinal axis or centerline defined by the elongate member. A second imager is disposed within the distal end portion of the elongate member and positioned substantially parallel to the first variable state optical member. The first imager produces an imaging signal based on optical energy reflected from tissue lateral to the elongate member when the second variable state optical member is in an optically-transmissive configuration. The first imager does not produce an imaging signal reflected from a tissue lateral to the elongate member when the second variable state optical member is in an optically-reflective or an optically-absorbing configuration. Optical energy from the distal aperture is not received by the first imager. Similarly, the second imager produces an imaging signal based on optical energy reflected from tissue distal to the elongate member when the first variable state optical member is in an optically-transmissive configuration. The second imager does not produce an imaging signal reflected from a tissue distal to the elongate member when the first variable state optical member is in an optically-reflective or an optically-absorptive configuration. Optical energy from the side aperture is not received by the second imager.

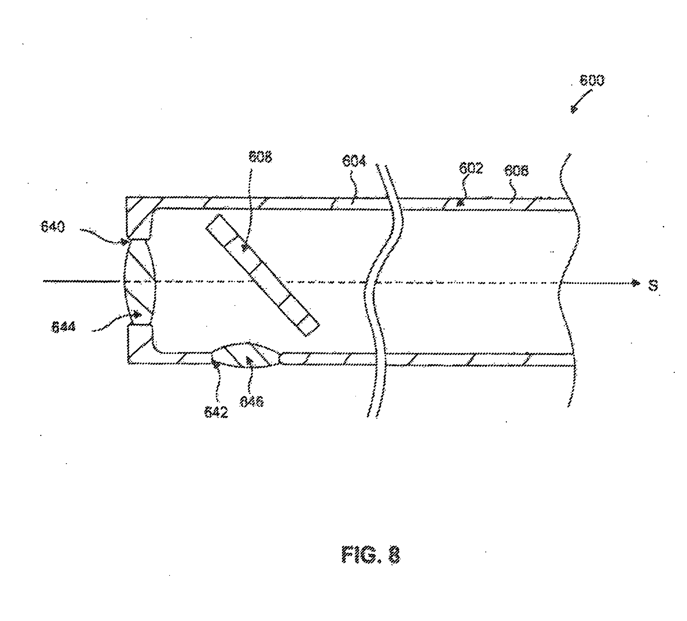

FIG. 8 illustrates a medical device according to another embodiment not part of the invention. An endoscope 600 may include an elongate member 602 having a distal end portion 604 and a proximal end portion 606. The distal end portion 604 of the elongate member 602 defines a distal aperture 640 and a side aperture 642. The distal end portion 604 of the elongate member 602 includes a first lens 644 and a second lens 646 fixedly disposed within the distal aperture 640 and the side aperture 642, respectively. The elongate member 602 defines an axis S. An imager 608 is disposed within the distal end portion 604 of the elongate member 602. Specifically, the imager 608 is positioned diagonally with respect to the axis J of the elongate member 602.

The first lens 644 and the second lens 646 each have a first configuration (e.g., an optically-absorptive configuration) and a second configuration (e.g., an optically-transmissive configuration). The imager 608 can produce an imaging signal associated with a tissue (not shown) distal to the elongate member 602 when the first lens 644 is in its second configuration and the second lens 646 is in its first configuration. The imager 608 can produce an imaging signal associated with a tissue (not shown) lateral to the elongate member 602 when the first lens 644 is in its first configuration and the second lens 646 is in its second configuration.

The first lens 644 absorbs optical energy, preventing the optical energy from passing through the first lens 644, when the first lens 644 is in its first configuration. The imager 608 can receive optical energy from the first lens 644 when the first lens 644 is in its second configuration. As a result, the first lens 644 prevents the imager 608 from receiving optical energy from the first lens 644 when the first lens 644 is in its first configuration and allows the imager 608 to receive optical energy from the first lens 644 when the first lens 644 is in its second configuration. Similarly, the second lens 646 absorbs optical energy, preventing the optical energy from passing through the second lens 646, when the second lens 646 is in its first configuration. The imager 608 can receive optical energy from the second lens 646 when the second lens 646 is in its second configuration. As a result, the second lens 646 prevents the imager 608 from receiving optical energy from the second lens 646 when the second lens 646 is in its first configuration and allows the imager 608 to receive energy from the second lens 646 when the second lens 646 is in the second configuration. In some embodiments, the first configuration is an optically-reflective or mirror-like configuration.

In some alternative embodiments not part of the invention, the imager can produce an imaging signal associated with tissue distal to elongate member 602 and can concurrently produce an imaging signal associated with tissue lateral to the elongate member 602. In such alternative embodiments, the imager can have a first imaging portion and a second imaging portion mutually exclusive from the first imaging portion. In such embodiments, the imager is positioned such that the first imaging portion receives optical energy from the first lens 644 and not optical energy from the second lens 646 when the first lens 644 and the second lens 646 are both in the second configuration. The imager is also positioned such that the second imagining portion receives optical energy from the second lens 646 and not the optical energy from the first lens 644 when the first lens 644 and the second lens 646 are both in the second configuration. As a result, the first imaging portion of the imager can produce an imaging signal associated with a tissue distal to the elongate member 602 when first lens 644 and the second lens 646 are both in the second configuration (e.g., a transparent configuration). Similarly, the second imaging portion of the imager can produce an imaging signal associated with a tissue lateral to the elongate member 602 when the first lens 644 and the second lens 646 are both in the second configuration (e.g., a transparent configuration).

In some embodiments not part of the invention, the lens has multiple portions where each portion has an optically-transmissive configuration and an optically-absorptive or optically reflective configuration.

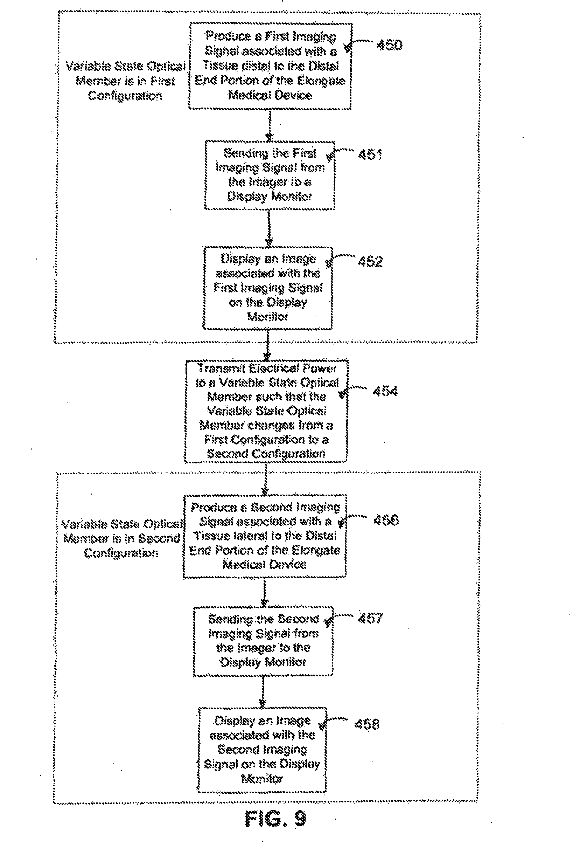

FIG. 9 is a flowchart illustrating a method of imaging a tissue using an endoscope according to an embodiment not part of the invention. At 450, an imager of an elongate medical device produces an imaging signal associated with at least a portion of a tissue distal to the elongate medical device. The elongate medical device can be, for example, an embodiment of an endoscope as described herein. For example, the elongate medical device can include an imager disposed within a distal end portion of the elongate medical device. The distal end portion of the elongate medical can be inserted into a patient. A variable state optical member is coupled within the distal end portion of the elongate medical device. The variable state optical member has a first configuration and a second configuration different than the first configuration. The imager can produce an imaging signal associated with a tissue distal to the elongate medical device when the variable state optical member in the first configuration. The imager can produce an imaging signal associated with a tissue lateral to the elongate medical device when the variable state optical member is in the second configuration.

The variable state optical member can be a first variable state optical member. The elongate medical device can optionally include a second variable state optical member that has a first configuration and a second configuration different from the first configuration. The imager can produce the imaging signal associated with the tissue distal to the elongate medical device when the first variable state optical member is in its first configuration and the second variable state optical member is in its first configuration. The imager can produce the imaging signal associated with the tissue lateral to the elongate medical device when the first variable state optical member is its second configuration and the second variable state optical member is in its second configuration.

At 451, the imaging signal associated with at least the portion of the tissue distal to the elongate medical device is sent from the imager to a display monitor. At 452, an image of the portion of the tissue distal to the elongate medical device is displayed at the display monitor. At 454, electrical power is transmitted to the variable state optical member such that the variable state optical member changes from the first configuration to the second configuration. At 456, an imaging signal associated with at least a portion of a tissue lateral to the elongate medical device is produced at the imager. At 457, the imaging signal associated with at least the portion of the tissue lateral to the elongate medical device is sent from the imager to the display monitor. At 458, an image of the portion of the tissue lateral to the elongate medical device is displayed at the display monitor.

In some embodiments not part of the invention, electrical power is transmitted to a second variable state optical member of the elongate medical device such that the second variable state optical member changes from a first configuration to a second configuration. In such embodiments, the imaging signal associated with the tissue distal to the elongate medical device is sent while the first variable state optical member is in the first configuration and the second variable state optical member is in the second configuration. The imaging signal associated with the tissue lateral to the elongate medical device is sent while the first variable state optical member is in the second configuration and the second state optical member is in the first configuration.

In some embodiments not part of the invention, the first variable state optical member is moved from a first position to a second position different than the first position. In such embodiments, the imager can send an imaging signal associated with a tissue distal and lateral to the elongate medical device. In some embodiments, a second variable state optical member of the elongate medical device is moved from a first position to a second position different from the first position.

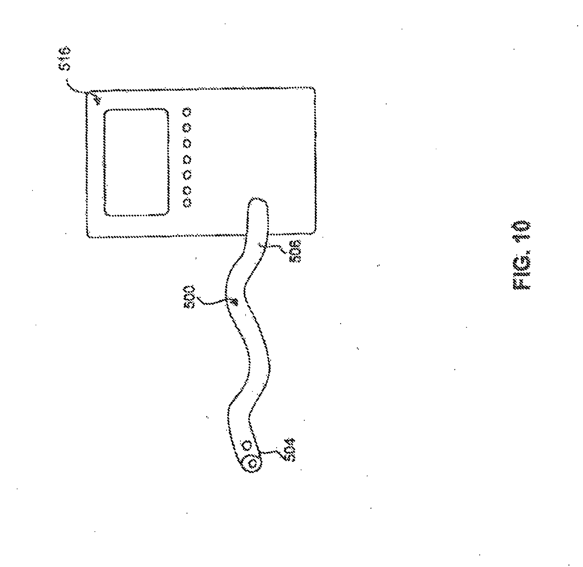

FIG. 10 is a side perspective view illustrating a medical device shown operatively coupled to a display monitor 516. An endoscope 500 is shown coupled to a display monitor 516 such that an image produced by a detector (not shown) at a distal end portion 504 of the endoscope 500 can be output to the display monitor 516. In some embodiments, a proximal end portion 506 of the endoscope 500 is operatively coupled to a controller (not shown), such as, for example, a computer, a processor, etc. In some embodiments, a handle (not shown) is coupled to the proximal end portion 506 of the endoscope 500. The handle can include at least one control device, such as, for example, an actuator to control and maneuver the endoscope 500.

One or more of the variable state optical members of the endoscope can be any of a variety of multiple configuration reflection components, including for example, a thermochromic mirror, a photochromic mirror, an electrochromic mirror, a transition-metal hydride electrochromic mirror, a solvatochromic mirror, a gasochromic mirror, a liquid crystal glass element, a polymer-dispersed liquid crystal glass element, metal hydride film mirror or the like. Specifically, glass has been developed that can change its light transmission properties due to, for example, an applied electrical potential (e.g., electrochromic), a change in the pressure of hydrogen (e.g., a gasochromic mirror), etc. For example, electrochromic glass can be used to attenuate the transmission of optical energy through the reflective surface and control the amount of reflectance of the glass. Liquid crystal glass can scatter incoming optical energy to prevent transmission of the optical energy through the glass. The metal hydride film mirror includes a metal hydride film that can optionally be alloyed with magnesium. For example, nickel-magnesium films, via cathodic polarization in alkaline electrolytes, uptake hydrogen causing the metallic mirror to become transparent. The variable state optical members can also include copper, copper oxides, pnicogens, lithium pnicogens, etc. In some embodiments, electrochromic glass (not mirrored) or anti-reflective coatings could be used to reduce unwanted reflection when in the transparent configuration.

Various manufacturers and/or entities associated with multiple configuration reflection components include, for example, Innovative Glass Corp., Smart Glass International and Polytronix, Inc., which produce liquid crystal glass. Similarly, transition-metal hydride electrochromics that change the light interaction characteristics of the reflection layer have been described in "Optical switches based on magnesium lanthanide alloy hydrides" Applied Physics Letter 70(25), 3356-3358, which is incorporated herein by reference in its entirety.

While various embodiments of the invention have been described above, it should be understood that they have been presented by way of example only, and not limitation. Various changes in form and details of the embodiments can be made.

For example, the endoscopes described herein can include various combinations and/or sub-combinations of the components and/or features of the different embodiments described. The endoscopes described herein can include one or more variable state optical members that also have multiple configurations.

An endoscope can have a variety of different shapes and sizes, and include a different quantity of lumens, and various different features and capabilities. For example, although the specific embodiments described herein illustrate an imager, other types of electrical components can alternatively be included.

## Claims

1. An apparatus, comprising:
an elongate member (102, 202) having a proximal end portion and a distal end portion (104, 204);
a variable state optical member (120, 220) disposed within the distal end portion of the elongate member (102, 202), the variable state optical member having a first configuration and a second configuration different from the first configuration, wherein the variable state optical member (120, 220) is configured to pass optical energy through the variable state optical member (120, 220) when the variable state optical member is in the second configuration: and
an imager (108, 208) disposed within the distal end portion of the elongate member (102, 202), the imager being configured to produce an imaging signal associated with a tissue distal to the elongate member (102, 202) when the variable state optical member (120, 220) is in the first configuration, and wherein the imager is further configured to produce an imaging signal associated with a tissue lateral to the elongate member when the variable state optical member is in the second configuration.

2. The apparatus of claim 1, wherein the variable state optical member (120, 220) is configured to reflect optical energy from the tissue distal to the elongate member when the variable state optical member (120, 220) is in the first configuration.

3. The apparatus of claim 1, wherein the variable state optical member (120, 220) is configured to absorb optical energy from the tissue lateral to the elongate member when the variable state optical member is in the first configuration.

4. The apparatus of claim 1, wherein the variable state optical member (120, 220) includes a first end portion and a second end portion opposite the first end portion, the imager (108, 208) and the first end portion of the variable state optical member (120, 220) being separated by a first distance, and the imager (108, 208) and the second end portion of the variable state optical member (120, 220) being separated by a second distance less than the first distance.

5. The apparatus of claim 1, wherein the distal end portion of the elongate member (102, 202) defines a distal aperture (140, 240) and a side aperture (142, 242), the apparatus further comprising:
a first lens (244) fixedly disposed within the distal aperture (140, 240) of the elongate member; and
a second lens (246) fixedly disposed within the side aperture (142, 242) of the elongate member.

6. The apparatus of claim 1, wherein the variable state optical member (120, 220) is configured to be moved between a first position and a second position different from the first position, and wherein the imager (108, 208) is configured to produce an imaging signal associated with a tissue disposed between the tissue distal to the elongate member (102, 202) and the tissue lateral to the elongate member (102, 202).

7. The apparatus of claim 1, further including:
an elongate conductive component (118, 218) electrically coupled to the variable state optical member (120, 220) and a power source (110).

8. The apparatus of claim 7, wherein the variable state optical member (120, 220) is configured to change from the first configuration to the second configuration upon application of power received from the power source (110).

9. The apparatus of claim 8, wherein the variable state optical member (120, 220) is configured to change from the second configuration to the first configuration when power is not received from the power source (110).

10. The apparatus of claim 7, wherein the elongate conductive component (118, 218) is electrically coupled to the imager (108, 208).

11. The apparatus of claim 1, wherein the variable state optical member (120, 220) is configured to change from the first configuration to the second configuration upon application of at least one of a gasochromic mechanism, a mechanical device, a shape changing mechanism, and an electroactive material.

12. The apparatus of claim 5, wherein one of the first lens (244) and the second lens (246) include at least one of a biconvex, plano-convex, convex-convex, meniscus, planoconcave, and biconcave shape.

13. The apparatus of claim 1, further including:
a processor (114) configured to receive information from the imager (108, 208).

14. The apparatus of claim 13, wherein the processor is further configured to deliver the received information to a display apparatus (116, 516).

## Patentansprüche

1. Einrichtung, umfassend:
ein längliches Element (102, 202), das einen proximalen Endabschnitt und einen distalen Endabschnitt (104, 204) aufweist;
ein optisches Element in variablem Zustand (120, 220), das innerhalb des distalen Endabschnitts des länglichen Elements (102, 202) angeordnet ist, wobei das optische Element in variablem Zustand eine erste Ausgestaltung und eine zweite Ausgestaltung, die sich von der ersten Ausgestaltung unterscheidet, aufweist, wobei das optische Element in variablem Zustand (120, 220) ausgestaltet ist, optische Energie durch das optische Element in variablem Zustand (120, 220) zu geben, wenn sich das optische Element in variablem Zustand in der zweiten Ausgestaltung befindet; und
einen Bildgeber (108, 208), der innerhalb des distalen Endabschnitts des länglichen Elements (102, 202) angeordnet ist, wobei der Bildgeber ausgestaltet ist, ein bildgebendes Signal zu erzeugen, das einem Gewebe distal zu dem länglichen Element (102, 202) zugehörig ist, wenn sich das optische Element in variablem Zustand (120, 220) in der ersten Ausgestaltung befindet, und wobei der Bildgeber überdies ausgestaltet ist, ein bildgebendes Signal zu erzeugen, das einem Gewebe lateral zu dem länglichen Element zugehörig ist, wenn sich das optische Element in variablem Zustand in der zweiten Ausgestaltung befindet.

2. Einrichtung nach Anspruch 1, wobei das optische Element in variablem Zustand (120, 220) ausgestaltet ist, optische Energie von dem Gewebe distal zu dem länglichen Element zu reflektieren, wenn sich das optische Element in variablem Zustand (120, 220) in der ersten Ausgestaltung befindet.

3. Einrichtung nach Anspruch 1, wobei das optische Element in variablem Zustand (120, 220) ausgestaltet ist, optische Energie von dem Gewebe lateral zu dem länglichen Element zu absorbieren, wenn sich das optische Element in variablem Zustand in der ersten Ausgestaltung befindet.

4. Einrichtung nach Anspruch 1, wobei das optische Element in variablem Zustand (120, 220) einen ersten Endabschnitt und einen zweiten Endabschnitt gegenüberliegend dem ersten Endabschnitt umfasst, wobei der Bildgeber (108, 208) und der erste Endabschnitt des optischen Elements in variablem Zustand (120, 220) durch einen ersten Abstand getrennt sind, und der Bildgeber (108, 208) und der zweite Endabschnitt des optischen Elements in variablem Zustand (120, 220) durch einen zweiten Abstand getrennt sind, der geringer als der erste Abstand ist.

5. Einrichtung nach Anspruch 1, wobei der distale Endabschnitt des länglichen Elements (102, 202) eine distale Öffnung (140, 240) und eine Seitenöffnung (142, 242) bestimmt, wobei die Einrichtung überdies umfasst:
eine erste Linse (244), die fest innerhalb der distalen Öffnung (140, 240) des länglichen Elements angeordnet ist; und
eine zweite Linse (246), die fest innerhalb der Seitenöffnung (142, 242) des länglichen Elements angeordnet ist.

6. Einrichtung nach Anspruch 1, wobei das optische Element in variablem Zustand (120, 220) ausgestaltet ist, zwischen einer ersten Position und einer zweiten Position, die sich von der ersten Position unterscheidet, bewegt zu werden, und wobei der Bildgeber (108, 208) ausgestaltet ist, ein bildgebendes Signal zu erzeugen, das einem Gewebe zugehörig ist, das zwischen dem Gewebe distal zu dem länglichen Element (102, 202) und dem Gewebe lateral zu dem länglichen Element (102, 202) angeordnet ist.

7. Einrichtung nach Anspruch 1, überdies umfassend:
ein längliches leitendes Bauteil (118, 218), das elektrisch an das optische Element in variablem Zustand (120, 220) und eine Stromquelle (110).gekoppelt ist.

8. Einrichtung nach Anspruch 7, wobei das optische Element in variablem Zustand (120, 220) ausgestaltet ist, bei Anlegen von Strom, der von der Stromquelle (110) erhalten wird, von der ersten Ausgestaltung in die zweite Ausgestaltung zu wechseln.

9. Einrichtung nach Anspruch 8, wobei das optische Element in variablem Zustand (120, 220) ausgestaltet ist, von der zweiten Ausgestaltung in die erste Ausgestaltung zu wechseln, wenn kein Strom von der Stromquelle (110) erhalten wird.

10. Einrichtung nach Anspruch 7, wobei das längliche leitende Bauteil (118, 218) elektrisch an den Bildgeber (108, 208) gekoppelt ist.

11. Einrichtung nach Anspruch 1, wobei das optische Element in variablem Zustand (120, 220) ausgestaltet ist, bei Anlegen mindestens eines gasochromen Mechanismus, einer mechanischen Vorrichtung, eines formändernden Mechanismus oder eines elektroaktiven Werkstoffs von der ersten Ausgestaltung in die zweite Ausgestaltung zu wechseln.

12. Einrichtung nach Anspruch 5, wobei eine, die erste Linse (244) oder die zweite Linse (246) mindestens eine bikonvexe, plankonvexe, konvex-konvexe, meniskusförmige, plankonkave oder bikonkave Form aufweist.

13. Einrichtung nach Anspruch 1, überdies umfassend:
einen Prozessor (114), der ausgestaltet ist, Informationen von dem Bildgeber (108, 208) zu empfangen.

14. Einrichtung nach Anspruch 13, wobei der Prozessor überdies ausgestaltet ist, die empfangenen Informationen an eine Anzeigeeinrichtung (116, 516) zu liefern.

## Revendications

1. Appareil, comprenant :
un élément allongé (102, 202) ayant une portion d'extrémité proximale et une portion d'extrémité distale (104, 204) ;
un élément optique à état variable (120, 220) disposé à l'intérieur de la portion d'extrémité distale de l'élément allongé (102, 202), l'élément optique à état variable ayant une première configuration et une deuxième configuration différente de la première configuration, dans lequel l'élément optique à état variable (120, 220) est configuré pour transmettre de l'énergie optique à travers l'élément optique à état variable (120, 220) quand l'élément optique à êtat variable est dans la deuxième configuration ; et
un imageur (108, 208) disposé à l'intérieur de la portion d'extrémité distale de l'élément allongé (102, 202), l'imageur étant configuré pour produire un signal d'imagerie associé à un tissu distal à l'élément allongé (102, 202) quand l'élément optique à état variable (120, 220) est dans la première configuration, et dans lequel l'imageur est également configuré pour produire un signal d'imagerie associé à un tissu latéral à l'élément allongé quand l'élément optique à état variable est dans la deuxième configuration.

2. Appareil selon la revendication 1, dans lequel l'élément optique à état variable (120, 220) est configuré pour réfléchir de l'énergie optique en provenance du tissu distal à l'élément allongé quand l'élément optique à état variable (120, 220) est dans la première configuration.

3. Appareil selon la revendication 1, dans lequel l'élément optique à état variable (120, 220) est configuré pour absorber de l'énergie optique en provenance du tissu latéral à l'élément allongé quand l'élément optique à état variable est dans la première configuration.

4. Appareil selon la revendication 1, dans lequel l'élément optique à état variable (120, 220) inclut une première portion d'extrémité et une deuxième portion d'extrémité en face de la première portion d'extrémité, l'imageur (108, 208) et la première portion d'extrémité de l'élément optique à état variable (120, 220) étant séparés par une première distance, et l'imageur (108, 208) et la deuxième portion d'extrémité de l'élément optique à état variable (120, 220) étant séparés par une deuxième distance inférieure à la première distance.

5. Appareil selon la revendication 1, dans lequel la portion d'extrémité distale de l'élément allongé (102, 202) définit un orifice distal (140, 240) et un orifice latéral (142, 242), l'appareil comprenant également :
une première lentille (244) disposée de façon fixe à l'intérieur de l'orifice distal (140, 240) de l'élément allongé ; et
une deuxième lentille (246) disposée de façon fixe à l'intérieur de l'orifice latéral (142, 242) de l'élément allongé.

6. Appareil selon la revendication 1, dans lequel l'élément optique à état variable (120, 220) est configuré pour être déplacé entre une première position et une deuxième position différente de la première position, et dans lequel l'imageur (108, 208) est configuré pour produire un signal d'imagerie associé à un tissu disposé entre le tissu distal à l'élément allongé (102, 202) et le tissu latéral à l'élément allongé (102, 202).

7. Appareil selon la revendication 1, incluant également :
un composant conducteur allongé (118, 218) couplé électriquement à l'élément optique à état variable (120, 220) et à une source d'alimentation électrique (110).

8. Appareil selon la revendication 7, dans lequel l'élément optique à état variable (120, 220) est configuré pour passer de la première configuration à la deuxième configuration lors de l'application d'une alimentation électrique reçue en provenance de la source d'alimentation électrique (110).

9. Appareil selon la revendication 8, dans lequel l'élément optique à état variable (120, 220) est configuré pour passer de la deuxième configuration à la première configuration si aucune alimentation électrique n'est reçue en provenance de la source d'alimentation électrique (110).

10. Appareil selon la revendication 7, dans lequel le composant conducteur allongé (118, 218) est couplé électriquement à l'imageur (108, 208).

11. Appareil selon la revendication 1, dans lequel l'élément optique à état variable (120, 220) est configuré pour passer de la première configuration à la deuxième configuration lors de l'application d'au moins un élément parmi un mécanisme gazochromique, un dispositif mécanique, un mécanisme à changement de forme et un matériau électro-actif.

12. Appareil selon la revendication 5, dans lequel une lentille parmi la première lentille (244) et la deuxième lentille (246) inclut au moins une forme parmi les formes biconvexes, plan-convexes, convexo-convexes, ménisques, plan-concaves et biconcaves.

13. Appareil selon la revendication 1, incluant également :
un processeur (114) configuré pour recevoir des informations en provenance de l'imageur (108, 208).

14. Appareil selon la revendication 13, dans lequel le processeur est également configuré pour délivrer les informations reçues à un appareil d'affichage (116, 516).
